(19) Europäisches Patentamt
European Patent Office
Office européen des brevets

(11) EP 1 602 638 A1

(12) EUROPÄISCHE PATENTANMELDUNG

(43) Veröffentlichungstag:
**07.12.2005 Patentblatt 2005/49**

(51) Int Cl.[7]: **C07C 41/58**, C07C 43/13

(21) Anmeldenummer: **05101847.1**

(22) Anmeldetag: **10.03.2005**

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**AL BA HR LV MK YU**

(30) Priorität: **29.04.2004 DE 102004021129**

(71) Anmelder: **Degussa AG**
**40474 Düsseldorf (DE)**

(72) Erfinder:
- **Köhler, Günther**
  **45770, Marl (DE)**
- **Brehme, Volker**
  **48155, Münster (DE)**
- **Neumann, Manfred**
  **45770, Marl (DE)**
- **Osterholt, Clemens**
  **46286, Dorsten (DE)**

(54) **Verfahren zur Isolierung von hochreinem 2-Methoxypropen**

(57) Die Erfindung betrifft ein Verfahren zur Isolierung von hochreinem 2-Methoxypropen aus Rohprodukten oder anderen Gemischen, die 2,2-Dimethoxypropan, 2-Methoxypropen, Methanol, Aceton und ggf. andere Carbonylverbindungen enthalten durch Destillation, wobei bei der Destillation 2-Aminoethanol und eine Base als Hilfsstoffe zugesetzt werden.

EP 1 602 638 A1

## Beschreibung

Die Erfindung betrifft ein Verfahren zur Isolierung von hochreinem 2-Methoxypropen aus Rohprodukten oder anderen Gemischen, die 2,2-Dimethoxypropan, 2-Methoxypropen, Methanol, Aceton und ggf. andere Carbonylverbindungen enthalten, welche beispielsweise bei der Synthese von 2-Methoxypropen anfallen.

2-Methoxypropen ist ein wertvolles Zwischenprodukt, das für die Synthese von Pharmawirkstoffen in hoher Reinheit benötigt wird. Für den Einsatz im Pharmabereich sind Reinheiten von > 99,0 % erforderlich und Grenzwerte von Verunreinigungen wie Carbonylverbindungen von max 0,2% insbesondere von max. 0,1 % einzuhalten.

2-Methoxypropen kann besonders vorteilhaft durch Pyrolyse von 2,2-Dimethoxypropan hergestellt werden, indem unter Methanolabspaltung ein Rohgemisch aus 2-Methoxypropen, Methanol und Aceton sowie einer Reihe weiterer Verunreinigungen gebildet wird. Die Herstellung kann sowohl batchweise als auch kontinuierlich ausgeführt werden, indem das Edukt 2,2-Dimethoxypropan mit einem Katalysator in Kontakt gebracht wird, der beispielsweise als Festbett oder als Kontaktzone vorliegt. Das Rohproduktgemisch wird im Falle der kontinuierlichen Fahrweise zeitgleich mit der Eduktzufuhr und der Reaktion aus der Reaktionszone entfernt. Herstellverfahren für 2-Methoxypropen über Pyrolyse von 2,2-Dimethoxypropan sind z. B. in EP 0 703 211 oder in EP 0 776 879, WO 2001/096269 oder Tetrahedron Letters 23 (6), 631-634, 1982 beschrieben.

Völlig andere Synthesewege zur Herstellung von 2-Methoxypropen gehen von 1-Propin oder Propen aus und werden z. B. in DE 102 33 231 oder in J.Am.Chem.Soc 1967, 89(18), 4684-7; beschrieben.

Das auf all diesen Wegen gewonnene 2-Methoxypropen erfüllt jedoch wegen der darin enthaltenen Verunreinigungen, insbesondere durch seinen Gehalt an Carbonylverbindungen wie z. B. Aceton keineswegs die Reinheits-Anforderungen für eine Pharmaanwendung. Einfache destillative Abtrennung ist mit vertretbarem technischem Aufwand nahezu unmöglich, da sich binäre, ternäre oder multiple Azeotrope bilden [Beregovykh, V.V.; Andrianove, O.N.; Babich, S.V.; Khimiko-Farmatsevticheskij Zhumal (1983), 17(4), 454-9] und extraktive Entfernung von Carbonylverbindungen wenig effektiv ist [Agre, B.A. et al. Vses. Nauchno-Issled. Inst. Org. Sint. Moscow Neftepererabtoka i Neftechimiya (Moscow) 1983, (1), 37-38].

Nach dem Stand der Technik sind eine Reihe von chemischen Reinigungsverfahren zur Nachbehandlung der Rohgemische bekannt, bei denen Spuren von Carbonylverbindungen aus der Synthese entfernt werden können.

So wird in der Patentschrift DE 332 823 ein Verfahren zur Entfernung von Verunreinigungen (Aldehyde und Ketone) durch die Nachbehandlung mit $NaBH_4$ beschrieben. Um 0,1 - 0,2 % von Verunreinigungen auf einen Wert von < 0,1 % zu minimieren, sind Mengen von ca. 10 Gew.-% $NaBH_4$ und 30 - 40 Gew.-% NaOH notwendig, was den Reinigungsaufwand entsprechend erhöht und das Produkt verteuert.

In einer weiteren Veröffentlichung werden zur Beseitigung von carbonylischen Verunreinigungen Diole hinzugefügt, die als Ketale bzw. Acetale derivatisiert werden und danach destillativ von den Wertprodukten abtrennbar sind, wie z. B in der US Patentschrift 3 578 568 erwähnt wird.

Die Patentschrift US 4 012 289 beschreibt ein weiteres Reinigungs-Verfahren zur Abtrennung von Acetonspuren durch Zugabe von Sulfolan.

In anderen Patenten, die die Reinigung von acetonhaltigen Produktgemischen beschreiben, werden Acetonspuren durch Luftoxidation in Gegenwart eines CuNi-Kontaktes auf Aluminiumoxidträger beseitigt (SU 728 902) und (SU 662 585).

Ein weiterer Weg zur Entfernung von Acetonspuren wird in der Patentschrift US 5 352 807 durch Behandlung mit Aktivkohle als Adsorbens beschrieben. Unter Nutzung von superkritischem $CO_2$ wird Aceton als Verunreinigung gemäß Patentschrift RU 2 110 523 extraktiv entfernt. Weitere Verfahren beschreiben die Entfernung von geringen Mengen Aceton aus Destillationsprodukten durch Behandlung mit Hydroxylamin (Patentschrift CS 109 181 und Levadie, Benjamin; Mac Askill, Stephen; Analytical Chemistry 1976, 48(11), 1656 ff.).

In US 5 271 835 wird ein Verfahren zur Entfernung von schwefelhaltigen Verunreinigungen aus Leichtölströmen durch Behandlung mit Alkanolaminen wie z. B. Methyldiethanolamin, Diethanolamin und Monoethanolamin beschrieben. Spuren an polaren Komponenten wie Aceton werden dort jedoch in der Hauptsache an einem festen Adsorbens entfernt, das anschließend wieder aufwendig regeneriert werden muss.

Gemäß US Patentschrift 3 607 003 kann Aceton aus Wasserstoffgasströmen, die Aceton als Verunreinigung enthalten, über einen Wäscher entfernt werden, der jedoch eine wässrige Monoethanolaminlösung enthält und bei Raumtemperatur betrieben wird.

In der Offenlegungsschrift DE 11 34 076 wird die Bildung von Schiffschen Basen aus Carbonylverbindungen und primären Aminen einschließlich Ethanolamin beschrieben, wobei erfindungsgemäß die Iminofunktion von einer Schiffschen Base einer bevorzugt niedermolekularen Carbonylverbindung auf die Carbonylfunktion einer zweiten Carbonylverbindung übertragen wird. Das Zielprodukt, die Schiff'sche Base der zweiten Carbonylverbindung, wird von der freigesetzten niedermolekularen Carbonylverbindung abgetrennt. Dies geschieht durch Auskristallisieren und ggf. Filtration, im Falle eines schwerlöslichen Reaktionsproduktes, oder Abdestillieren der neu gebildeten Schiffschen Base oder vorzugsweise durch Abdestillieren der niedermolekularen Carbonylverbindung. Ein vollständiges Zurückhalten

der Carbonylverbindung im Sumpf der Destillation wird hier nicht beschrieben.

Alle die nach dem Stand der Technik bekannten Verfahren haben entweder den Nachteil, dass sie technisch sehr aufwendig sind - wie z. B. in der o.g. Patentschrift DE 11 34 076 - oder den Grad der erforderlichen Reinigung bzw. die Effizienz der Entfernung von Carbonylverbindungen für eine Pharmaanwendung von 2-Methoxypropen nicht erfüllen, bei der es darauf ankommt eine Höchstgrenze von max. 0,2 % vorzugsweise jedoch max. 0,1 % Aceton nicht zu überschreiten.

Es bestand daher die Aufgabe, ein möglichst einfaches im technischen Maßstab durchführbares Verfahren zur Isolierung von 2-Methoxypropen aus 2-Methoxypropen enthaltenden Gemischen bereitzustellen, welches sehr gute Ausbeuten ermöglicht und ein hochreines Produkt zur Verfügung stellt, welches maximal 0,2 %, vorzugsweise jedoch max. 0,1 % Carbonylverbindung, insbesondere Aceton, enthalten darf. Es sollten dabei vor allem Rohprodukte oder Gemische als Einsatzmaterial eingesetzt werden können, die neben 2,2-Dimethoxypropan als Verunreinigungen Methanol, Aceton und auch weitere Carbonylverbindungen enthalten können. Insbesondere sollten Reaktionsausträge der 2-Methoxypropensynthese eingesetzt werden können. Das Verfahren sollte möglichst ohne großen apparativen Mehraufwand durchführbar sein und es sollten dabei insbesondere die Nachteile der herkömmlichen Verfahren vermieden werden.

Gelöst werden diese sowie weitere nicht explizit genannten Aufgaben, die jedoch aus den hierin diskutierten Zusammenhängen ohne weiteres ableitbar oder erschließbar sind durch ein Verfahren nach Anspruch 1. Zweckmäßige Ausformungen und Abwandlungen des erfindungsgemäßen Verfahrens werden in den auf Anspruch 1 rückbezogenen Unteransprüchen unter Schutz gestellt.

Dadurch dass man, ein Destillationsverfahren zur Isolierung von hochreinem 2-Methoxypropen aus Rohprodukten oder anderen Gemischen, die 2,2-Dimethoxypropan, 2-Methoxypropen, Methanol, Aceton und ggf. andere Carbonylverbindungen enthalten, anwendet, bei dem bei der Destillation 2-Aminoethanol und eine Base als Hilfsstoffe zugesetzt werden, gelingt es, ein hochreines Produkt, welches maximal 0,2 %, vorzugsweise jedoch max. 0,1 % Carbonylverbindung, insbesondere Aceton, enthält, in sehr guten Ausbeuten zur Verfügung zu stellen und die Nachteile der bekannten Reinigungsverfahren zu überwinden.

Dabei werden bei Verwendung von Reaktionsausträgen der 2-Methoxypropensynthese diese vor der Destillation in üblicher Weise zunächst mit Wasser extrahiert zur Abtrennung der Hauptmenge an wasserlöslichen Nebenprodukten, z.B. des Acetons.

Während der anschließenden erfindungsgemäßen Destillation mit Zusatz von 2-Aminoethanol und einer Base als Hilfsstoff bilden sich die entsprechenden Schiff'schen Basen aus Carbonylverbindung und 2-Aminoethanol. Dabei ist die destillative Abtrennung des Reaktionswassers aus der Schiff-Base-Reaktion als spezieller Verfahrensschritt nicht erforderlich.

Erfindungsgemäß bevorzugt wird dabei mindestens eine Base eingesetzt, ausgewählt aus der Gruppe bestehend aus Alkalimetallhydroxid, Erdalkalimetallhydroxid, Alkalimetallcarbonat, Erdalkalimetallcarbonat, Alkalimetallhydrogencarbonat, Erdalkalimetallhydrogencarbonat, Alkalimetallalkoholat, Erdalkalimetallalkoholat, Alkalimetallcarboxylat bzw. Erdalkalimetallcarboxylat einer ein- oder mehrbasigen Carbonsäure mit mindestens 2 C-Atomen und organische N-haltige Base.

Dabei werden als Alkalimetall bzw. Erdalkalimetall vorzugsweise Lithium, Natrium, Kalium, Rubidium, Cäsium, Magnesium, Calcium, Strontium oder Barium verwendet.

Als Alkalimetallcarboxylat bzw. Erdalkalimetallcarboxylat einer ein- oder mehrbasigen Carbonsäure mit mindestens 2 C-Atomen werden dabei bevorzugt Carboxylate der gesättigten $C_2$- bis $C_{18}$-Carbonsäuren, der gesättigten $C_2$- bis $C_6$-Dicarbonsäuren, der monohydroxysubstituierten $C_2$- bis $C_6$-Mono-, di- oder tricarbonsäuren eingesetzt.

Als Alkalimetallalkoholat, Erdalkalimetallalkoholat werden bevorzugt Alkoholate der linearen oder verzweigten aliphatischen $C_1$- bis $C_8$-Alkohole verwendet.

Als organische N-haltige Base werden bevorzugt eine oder mehrere Verbindungen der allgemeinen Formel I, II, III, IV, V, VI oder VII verwendet,

Formel I:

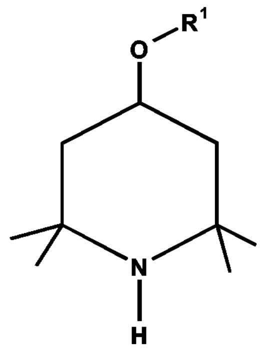

wobei $R^1$ Wasserstoff, ein aliphatischer oder cycloaliphatischer, gesättigter bzw. ungesättigter Kohlenwasserstoffrest mit 1 bis 18 C-Atomen, ein gesättigter bzw. ungesättigter linearer oder verzweigter Acylrest oder ein Säurerest einer gesättigten $C_3$- bis $C_{20}$-Dicarbonsäure bzw. einer ungesättigten $C_4$- bis $C_{20}$-Dicarbonsäure oder eine Polyacylverbindung oder eine Polysiloxanylverbindung der Formel

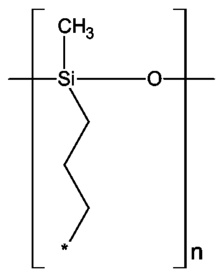

ist, wobei das Symbol Stern für das Sauerstoffatom in Formel I steht und
n= 2 bis 10 ist,

Formel II:

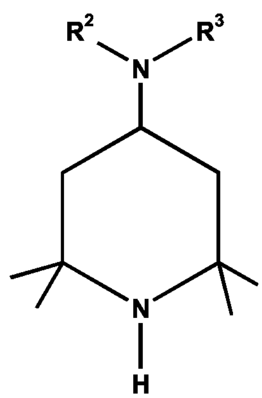

wobei $R^2$ und $R^3$ gleich oder verschieden sein können und Wasserstoff, einen aliphatischen oder cycloaliphatischen, gesättigten bzw. ungesättigten Kohlenwasserstoffrest mit 1 bis 18 C-Atomen bedeuten oder eine von beiden eine gesättigte oder ungesättigte gegebenenfalls alkylsubstituierte Alkylenkette mit 2 bis 18 C-Atomen bedeutet, die mit dem N-Atom eines zweiten Moleküls der Formel II verbunden ist, oder $R^2$ und $R^3$ für einen am N-Atom gebundenen Säurerest einer aliphatischen, gesättigten oder ungesättigten $C_4$- bis $C_5$-Dicarbonsäure, die an einem oder mehreren C-Atomen $C_1$- bis $C_{18}$-alkylsubstituiert sein kann, oder für einen am N-Atom gebundenen Säurerest einer aromatischen 1,2-Dicarbonsäure steht,

Formel III:

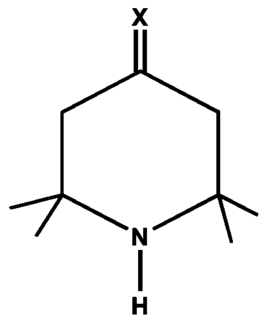

wobei X = O oder N-$R^4$ ist und $R^4$ ein aliphatischer oder cycloaliphatischer, gesättigter oder ungesättigter Kohlenwasserstoffrest mit 1 bis 18 C-Atomen ist,

Formel IV:

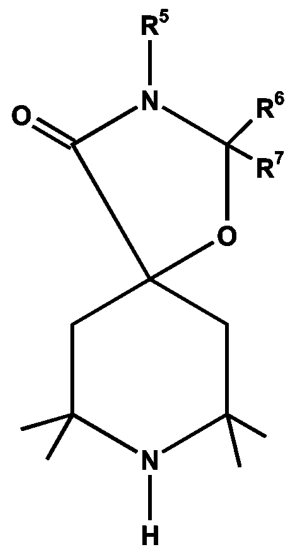

wobei $R^5$, $R^6$ und $R^7$ gleich oder verschieden sein können und Wasserstoff oder einen aliphatischen oder cycloaliphatischen, gesättigten bzw. ungesättigten Kohlenwasserstoffrest mit 1 bis 18 C-Atomen bedeuten oder $R^6$ und $R^7$ für eine aliphatische, gesättigte oder ungesättigte gegebenenfalls verzweigte Alkylenkette $-(CH_2)_m-$ mit m = 2 bis 18 steht,

Formel V: $NR^8R^9R^{10}$

wobei $R^8$, $R^9$ und $R^{10}$ gleich oder verschieden sein können und einen aliphatischen, gesättigten oder ungesättigten $C_1$- bis $C_{18}$-Alkylrest oder einen aliphatischen, gesättigten oder ungesättigten $C_5$- bis $C_{18}$-Cycloalkylrest bedeuten und maximal zwei Reste gleichzeitig $C_1$-Alkyl sein dürfen,

Formel VI: $HNR^8R^9$

wobei $R^8$ und $R^9$ die oben angegebene Bedeutung haben und dabei maximal ein Rest $C_1$-Alkyl sein darf,

Formel VII: $NR^8R^9R^{10}R^{11}OH$

wobei $R^8$, $R^9$, $R^{10}$, $R^{11}$ gleich oder verschieden sein können und $R^8$, $R^9$, $R^{10}$ die oben genannte Bedeutung haben und $R^{11}$ ebenfalls ein gesättigter oder ungesättigter $C_1$- bis $C_{18}$-Alkylrest oder ein aliphatischer, gesättigter oder ungesättigter $C_5$- bis $C_{18}$-Cycloalkylrest ist.

Ganz besonders bevorzugt werden als N-haltige Base jedoch Verbindungen der vorstehend aufgeführten Formeln I, II, III, IV, V, VI oder VII verwendet, bei denen

| | |
|---|---|
| $R^1$ = | Acrylolyl, Methacryloyl, 2-Alkylacryloyl mit einer $C_2$- bis $C_6$-Alkylgruppe, Succinyl-, Maleinoyl-, Polyacryloyl-, der Säurerest eines Copolymeren aus Ethylen und Acrylsäure oder Hexamethylenbiscarbonyl, |
| $R^2$, $R^3$ = | n-Butyl, |
| $R^2$ + $R^3$ = | Säurerest der Bernsteinsäure, der Glutarsäure oder der Phthalsäure, |
| $R^5$ = | H und m = 11, |

$R^8$, $R^9$, $R^{10}$ in Formel V Ethyl,
$R^8$, $R^9$, $R^{10}$, $R^{11}$ in Formel VII Methyl, Ethyl oder Propyl ist.

Darüber hinaus ganz ausserordentlich bevorzugt werden als N-haltige Base 3-Dodecyl-1-(2,2,6,6-Tetramethyl-4-piperidyl)pyrrolidin-2,5-dion (S95), Diazabicyclooctan oder 4-Dimethylaminopyridin eingesetzt.

Erfindungsgemäß bevorzugt werden 2-Aminoethanol und die Base während der Destillation gleichzeitig, nacheinander oder intermittierend dem Destillationssumpf oder der Rohstoffeinspeisung für die Destillation zugefügt. Die Rohstoffeinspeisung kann dabei wahlweise in den oberen, mittleren oder unteren Teil der Destillationskolonne erfolgen.

Die Menge des hinzugefügten Aminoethanols ist abhängig von der Menge an enthaltenen Verunreinigungen nämlich der Carbonylverunreinigungen. Erfindungsgemäß wird dabei 2-Aminoethanol vorzugsweise in einem Verhältnis von 1 bis 10 Moläquivalenten, besonders bevorzugt in einem Verhältnis von 1,2 bis 5 Moläquivalenten, insbesondere von 1,5 bis 3 Moläquivalenten bezogen auf den Gehalt an Carbonylverbindung eingesetzt.

Als bevorzugte Menge an Base wird ein Anteil von 0,5 bis 5 Gew.-%, besonders bevorzugt ein Anteil von 1 - 4 Gew.-% bezogen auf die Einsatzmenge der Destillation eingesetzt.

Erfindungsgemäß wird während der Destillation vorzugsweise eine Sumpftemperatur von 30 bis 100 °C, insbesondere von 50 bis 80 °C gewählt, sowie ein Druck, der vorzugsweise im Bereich von 100 mbar bis 5 bar , insbesondere jedoch von 900 mbar bis 4 bar, aber ganz besonders bevorzugt bei Normaldruck liegt.

Das erfindungsgemäße Destillationsverfahren kann sowohl kontinuierlich aber auch diskontinuierlich durchgeführt werden

Erfindungsgemäß bevorzugt werden zur Destillation Gemische eingesetzt, die aus der Synthese von 2-Methoxypropen resultieren.

Überraschenderweise lässt sich mit Hilfe des erfindungsgemäßen Verfahrens so auf destillativem Wege hochreines 2-Methoxypropen herstellen. Es können Reinheiten > 99,0 % 2-Methoxypropen und Verunreinigungen an Carbonylverbindungen von in Summe < 0,1 % mühelos erzielt werden.

Das Verfahren ist sehr einfach in seiner Durchführung, da im ersten Aufarbeitungsschritt zunächst in übliche Anlagen wie Gegenstromextraktionskolonnen oder Rührkesseln das Rohgemisch mit extrahiert wird. Dabei wird in herkömmlicher Weise Methanol extraktiv entfernt. Im zweiten eigentlichen Aufarbeitungsschritt wird das erhaltene praktisch methanolfreie Gemisch in eine Destillationsvorrichtung, vorzugsweise in den Destillationssumpf eingetragen und das Rohgemisch gemeinsam mit Aminoethanol und einer Base destillativ aufgearbeitet.

Erfindungsgemäß werden in der Technik übliche kontinuierlich oder batchweise arbeitende Destillationsanlagen mit vorzugsweise ca. 5- 20 theoretischen Trennstufen verwendet, um das eingesetzte Gemisch destillativ aufzuarbeiten. Dabei werden in der Regel mit dem Vorlauf Spuren von niedrigsiedenden Verunreinigungen, mit dem Hauptlauf das hochreine 2-Methoxypropen und mit dem Nachlauf geringe Mengen Wasser und höhersiedende Komponenten wie z. B. 2,2-Dimethoxypropan destillativ voneinander getrennt.

In der Praxis haben sich zur Durchführung der letztgenannten Destillation bevorzugt Verfahrensausführungen in Destillationskolonnen mit strukturierten Destillationspackungen bewährt. Gute Ergebnisse werden z. B. mit einer Kolonne von ca. 10 bis 15 theoretischen Trennstufen erzielt.

Als hochsiedender Destillationsrückstand werden die Schiff'sche Base und weitere Hochsieder inklusive der ebenfalls zugefügten Base zurückbehalten. Aus diesem kann wahlweise sowohl die Base als auch die Schiff'sche Base und aus dieser der gebundene 2-Aminoethanol durch die üblichen dem Fachmann bekannten Methoden zurückgewonnen werden und ggf. direkt oder nach weiterer Reinigung im Verfahren erneut eingesetzt werden.

Ein großer Vorteil des erfindungsgemäßen Verfahrens ist, dass es mit den in der Technik üblichen Vorrichtungen durchführbar ist und darüber hinaus nur vergleichsweise geringe Mengen an Hilfsstoffen eingesetzt werden müssen, die zudem noch leicht abgetrennt und ggf. regeneriert und recycliert werden können.

Insbesondere die Trennung der bei destillativer Isolierung von 2-Methoxypropen auftretenden komplexen Azeotrope wird erfindungsgemäß auf besonders einfache Art ermöglicht. Gleichzeitig wird die sonst so gefürchtete

Bildung von Hydroperoxiden während der Destillation des Enolethers durch die Basizität des Systems zurückgedrängt. Darüber hinaus wird das Wasser, welches im Laufe der Destillation eine Rückspaltung von 2-Methoxypropen zu Aceton und Methanol bewirken könnte, durch das Verfahren im Sumpf bzw. im Nachlauf der Destillation zurückgehalten.

Durch das erfindungsgemäße Verfahren wird eine destillative Reinigung von 2-Methoxypropen mit einem wirtschaftlich vertretbaren Aufwand und den o.g. hohen Reinheitsgraden für Pharmaanwendungen überhaupt erst ermöglicht.

Die im Folgenden aufgeführte Beispiele soll die Erfindung weiter verdeutlichen aber nicht einschränken.

**Beispiel 1**

Nach in herkömmlicher Weise erfolgter wässriger Extraktion eines Reaktionsgemisches, das aus der Synthese von 2-Methoxypropen ausgehend von 2,2-Dimethoxypropan z. B. gemäß EP 0 703 211 stammt, liegt eine Rohgemisch vor, das beispielsweise eine Zusammensetzung folgender Komponenten hat:

| | |
|---|---|
| Wasser | 0,13 Gew.-% |
| Methanol | 0,14 Gew.-% |
| Aceton | 2,64 Gew.-% |
| 2-Methoxypropen (MOP) | 65,6 Gew.-% |
| 2,2-Dimethoxypropan (DMP) | 31,5 Gew.-% |

In den Destillationssumpf der Reindestillation (Sumpf) wurden ca. 5 Gew.-% Aminoethanol und ca. 2 Gew.-% 3-Dodecyl-1-(2,2,6,6-Tetramethyl-4-piperidyl)pyrrolidin-2,5-dion (S 95, CAS 79720-19-7) als Base hinzugefügt. Diese Mischung wurde mit Hilfe einer Destillationskolonne mit 15 theoretischen Trennstufen fraktioniert destilliert und gaschromatographisch die Zusammensetzungen ermittelt.

**Destillation :**

| | Masse | Wasser | MeOH | Aceton | 2-MOP | 2,2-DMP |
|---|---|---|---|---|---|---|
| | [kg ] | (Gehalt in Flächen-% *) | | | | |
| **Rohgemisch** | 15 | | | | | |
| **Zusatz S 95** | 0,30 | | | | | |
| **Zusatz 2-Amino-ethanol** | 0,75 | | | | | |
| **Sumpf** | 15 | 0,13 | 0,14 | 2,64 | 65,56 | 31,53 |
| Fraktion 1 | 0,80 | 0,01 | 1,07 | 0,04 | 57,45 | 40,77 |
| Fraktionen 2-9 | 8,5 | 0,06 | 0,01 | 0,05 | 99,8 | <0,01 |
| Fraktion 10 | 4,90 | 0,37 | 0,03 | 0,18 | 1,28 | 98,01 |
| Rückstand | 1,80 | | | | | |

*) Flammenionisationsdetektor

| Bedingungen : | | | | |
|---|---|---|---|---|
| | Temperaturen in [°C] | | Rücklauf-Verh. | Druck |
| Fraktionen | Sumpf | Kopf | R:A[1)] | mbar |
| | | | | |
| 1 | 53 | 34 | 1 : 1 | 1000 |

1)R:A = Rücklauf: Abnahmemenge [in Volumenteilen]

(fortgesetzt)

| Bedingungen : | | | | |
|---|---|---|---|---|
| | Temperaturen in [°C] | | Rücklauf-Verh. | Druck |
| Fraktionen | Sumpf | Kopf | R:A[1)] | mbar |
| 2-9 | 59 | 35 | 6 : 1 | 1000 |
| 10 | 65 | 35 | 6 : 1 | 1000 |

1)R:A = Rücklauf: Abnahmemenge [in Volumenteilen]

Aus 15 kg Einsatzprodukt, die theoretisch 9,8 kg 2-Methoxypropen enthielten, wurden 8,5 kg hochreines 2-Methoxypropen mit einem Gehalt von 99,8 % und Verunreinigungen an Aceton, Methanol sowie 2,2-DMP < 0,1 % erhalten. Dies entspricht einer Destillationsausbeute bezogen auf 2-MOP von 87 %.

**Beispiel 2**

Anstelle der im Beispiel 1 beschriebenen stickstoffhaltigen Base wurden 2 Gew.- % 4-Amino-2,2,6,6-Tetramethylpiperidin eingesetzt. Die ansonsten gleiche Mischung wie unter Beispiel 1 wurde wiederum in Gegenwart von Aminoethanol mit Hilfe einer Destillationskolonne mit 15 theoretischen Trennstufen fraktioniert destilliert.

**Destillation :**

| | Masse | Wasser | MeOH | Aceton | 2-MOP | 2,2-DMP |
|---|---|---|---|---|---|---|
| | [kg ] | (Gehalt in Flächen-% *) | | | | |
| **Rohgemisch** | 15 | | | | | |
| **Zusatz 1 :** **4- Amino- 2,2,6,6-Tetramethylpiperidin** | 0,9 | | | | | |
| **Zusatz 2-Aminoethanol** | 0,3 | | | | | |
| **Sumpf** | 15 | 0,15 | 0,11 | 2,7 | 65,9 | 31,4 |
| Fraktion 1 | 1,1 | 0,06 | 1,2 | 0,1 | 54,4 | 39,8 |
| Fraktionen 2-9 | 7,9 | 0,03 | 0,01 | 0,09 | 99,7 | <0,01 |
| Fraktion 10 | 5,5 | 0,01 | 0,01 | 0,2 | 1,4 | 98,2 |
| Rückstand | 1,2 | | | | | |

*) Flammenionisationsdetektor

| Bedingungen : | | | | |
|---|---|---|---|---|
| | Temperaturen in [°C] | | Rücklauf-Verh. | Druck |
| Fraktionen | Sumpf | Kopf | R:A[1)] | mbar |
| | | | | |
| 1 | 53 | 34 | 1 : 1 | 1000 |
| 2-9 | 60 | 35 | 6 : 1 | 1000 |

1) R:A = Rücklauf: Abnahmemenge [in Volumenteilen]

(fortgesetzt)

| Bedingungen : | | | | |
|---|---|---|---|---|
| | Temperaturen in [°C] | | Rücklauf-Verh. | Druck |
| Fraktionen | Sumpf | Kopf | R:A[1)] | mbar |
| 10 | 65 | 35 | 6 : 1 | 1000 |

1) R:A = Rücklauf: Abnahmemenge [in Volumenteilen]

Aus 15 kg Einsatzprodukt, die theoretisch 9,8 kg 2-Methoxypropen enthielten, wurden 8,5 kg hochreines 2-Methoxypropen mit einem Gehalt von 99,7 % und Verunreinigungen an Aceton, Methanol sowie 2,2-DMP ≤ 0,1% erhalten. Dies entspricht einer Destillationsausbeute bezogen auf 2-MOP von 80,4 %. Der Etherperoxidgehalt im Hauptlauf war < 10 ppm.

**Beispiel 3**

Anstelle der in Beispiel 1 beschriebenen Mischung wurde in Gegenwart der Zusätze 2-Aminoethanol und $K_2CO_3$ destilliert. Die ansonsten gleiche Mischung wie unter Beispiel 1 wurde wiederum mit Hilfe einer Destillationskolonne mit 15 theoretischen Trennstufen fraktioniert destilliert.

**Destillation :**

| | Masse | Wasser | MeOH | Aceton | 2-MOP | 2,2-DMP |
|---|---|---|---|---|---|---|
| | [kg ] | (Gehalt in Flächen-% *) | | | | |
| **Rohgemisch** | 15 | | | | | |
| **Zusatz 1 :** 4- Amino- 2,2,6,6-Tetramethylpiperidin | 0,9 | | | | | |
| **Zusatz 2-Aminoethanol** | 0,3 | | | | | |
| **Sumpf** | 15 | 0,15 | 0,11 | 2,7 | 65,9 | 31,4 |
| Fraktion 1 | 1,1 | 0,06 | 1,2 | 0,1 | 54,4 | 39,8 |
| Fraktionen 2-9 | 7,9 | 0,03 | 0,01 | 0,09 | 99,7 | <0,01 |
| Fraktion 10 | 5,5 | 0,01 | 0,01 | 0,2 | 1,4 | 98,2 |
| Rückstand | 1,2 | | | | | |

*) Flammenionisationsdetektor

| Bedingungen : | | | | |
|---|---|---|---|---|
| | Temperaturen in [°C] | | Rücklauf-Verh. | Druck |
| Fraktionen | Sumpf | Kopf | R:A[1)] | mbar |
| | | | | |
| 1 | 53 | 34 | 1 : 1 | 1000 |
| 2-9 | 60 | 35 | 6 : 1 | 1000 |

1) R:A = Rücklauf: Abnahmemenge [in Volumenteilen]

(fortgesetzt)

| Bedingungen : | | | | |
|---|---|---|---|---|
| | Temperaturen in [°C] | | Rücklauf-Verh. | Druck |
| Fraktionen | Sumpf | Kopf | R:A[1)] | mbar |
| 10 | 65 | 35 | 6 : 1 | 1000 |

1) R:A = Rücklauf: Abnahmemenge [in Volumenteilen]

Aus 15 kg Einsatzprodukt, die theoretisch 9,8 kg 2-Methoxypropen enthielten, wurden 7,4 kg hochreines 2-Methoxypropen (Fraktion 2-9) mit einem Gehalt von 99,4 % erhalten. Dies entspricht einer Destillationsausbeute bezogen auf 2-MOP von 75,1 %. Der Etherperoxidgehalt im Hauptlauf betrug < 20 ppm.

**Patentansprüche**

1. Verfahren zur Isolierung von hochreinem 2-Methoxypropen aus Rohprodukten oder anderen Gemischen, die 2,2-Dimethoxypropan, 2-Methoxypropen, Methanol, Aceton und ggf. andere Carbonylverbindungen enthalten, durch Destillation,
**dadurch gekennzeichnet,**
**dass** bei der Destillation 2-Aminoethanol und eine Base als Hilfsstoffe zugesetzt werden.

2. Verfahren nach Anspruch 1,
**dadurch gekennzeichnet,**
**dass** mindestens eine Base ausgewählt aus der Gruppe bestehend aus
Alkalimetallhydroxid, Erdalkalimetallhydroxid,
Alkalimetallcarbonat, Erdalkalimetallcarbonat,
Alkalimetallhydrogencarbonat, Erdalkalimetallhydrogencarbonat,
Alkalimetallalkoholat, Erdalkalimetallalkoholat,
Alkalimetallcarboxylat bzw. Erdalkalimetallcarboxylat
einer ein- oder mehrbasigen Carbonsäure mit mindestens 2 C-Atomen und organische N-haltige Base eingesetzt wird.

3. Verfahren nach Anspruch 2,
**dadurch gekennzeichnet,**
**dass** als Alkalimetall bzw. Erdalkalimetall Lithium, Natrium, Kalium, Rubidium, Cäsium, Magnesium, Calcium, Strontium oder Barium verwendet werden.

4. Verfahren nach einem der Ansprüche 2 oder 3,
**dadurch gekennzeichnet,**
**dass** Carboxylate der gesättigten $C_2$- bis $C_{18}$-Carbonsäuren, der gesättigten $C_2$- bis $C_6$-Dicarbonsäuren, der monohydroxysubstituierten $C_2$- bis $C_6$-Mono-, Di- oder Tricarbonsäuren eingesetzt werden.

5. Verfahren nach einem der Ansprüche 2 bis 3,
**dadurch gekennzeichnet,**
**dass** Alkoholate der linearen oder verzweigten aliphatischen $C_1$- bis $C_8$-Alkohole verwendet werden.

6. Verfahren nach Anspruch 2,
**dadurch gekennzeichnet,**
**dass** als organische N-haltige Base eine oder mehrere Verbindungen der allgemeinen Formel I, II, III, IV, V, VI oder VII verwendet werden,

Formel I:

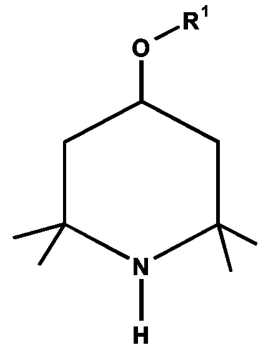

wobei $R^1$ Wasserstoff, ein aliphatischer oder cycloaliphatischer, gesättigter bzw. ungesättigter Kohlenwasserstoffrest mit 1 bis 18 C-Atomen, ein gesättigter bzw. ungesättigter linearer oder verzweigter Acylrest oder ein Säurerest einer gesättigten $C_3$- bis $C_{20}$-Dicarbonsäure bzw. einer ungesättigten $C_4$- bis $C_{20}$-Dicarbonsäure oder eine Polyacylverbindung oder eine Polysiloxanylverbindung der Formel

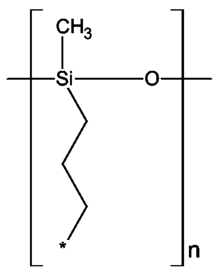

ist, wobei das Symbol Stern für das Sauerstoffatom in Formel I steht und
n= 2 bis 10 ist,

Formel II:

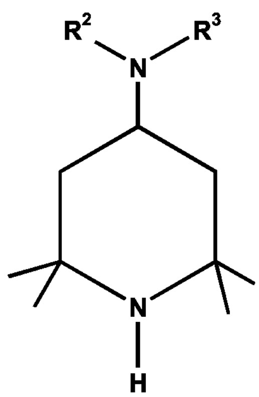

wobei $R^2$ und $R^3$ gleich oder verschieden sein können und Wasserstoff, einen aliphatischen oder cycloaliphatischen, gesättigten bzw. ungesättigten Kohlenwasserstoffrest mit 1 bis 18 C-Atomen bedeuten oder eine von beiden eine gesättigte oder ungesättigte gegebenenfalls alkylsubstituierte Alkylenkette mit 2 bis 18 C-Atomen bedeutet, die mit dem N-Atom eines zweiten Moleküls der Formel II verbunden ist, oder $R^2$ und $R^3$ für einen am N-Atom gebundenen Säurerest einer aliphatischen, gesättigten oder ungesättigten $C_4$- bis $C_5$-Dicarbonsäure, die an einem oder mehreren C-Atomen $C_1$- bis $C_{18}$-alkylsubstituiert sein kann oder für einen am N-Atom gebundenen Säurerest einer aromatischen 1,2-Dicarbonsäure steht,

Formel III:

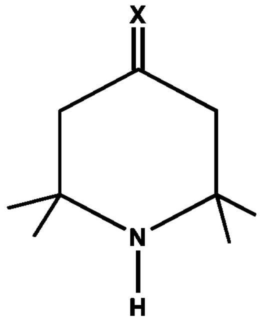

wobei X = O oder N-$R^4$ ist und $R^4$ ein aliphatischer oder cycloaliphatischer, gesättigter oder ungesättigter Kohlenwasserstoffrest mit 1 bis 18 C-Atomen ist,

Formel IV:

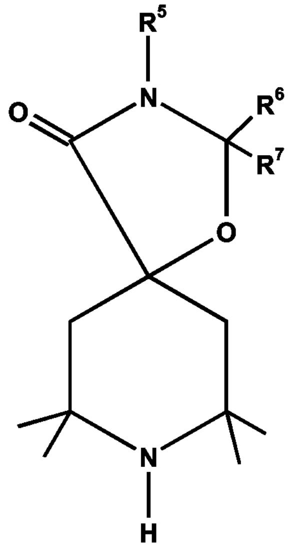

wobei $R^5$, $R^6$ und $R^7$ gleich oder verschieden sein können und Wasserstoff oder einen aliphatischen oder cycloaliphatischen, gesättigten bzw. ungesättigten Kohlenwasserstoffrest mit 1 bis 18 C-Atomen bedeuten oder $R^6$ und $R^7$ für eine aliphatische, gesättigte oder ungesättigte gegebenenfalls verzweigte Alkylenkette $-(CH_2)_m-$ mit m = 2 bis 18 steht,

Formel V: $NR^8R^9R^{10}$

wobei $R^8$, $R^9$ und $R^{10}$ gleich oder verschieden sein können und einen aliphatischen, gesättigten oder ungesättigten $C_1$- bis $C_{18}$-Alkylrest oder einen aliphatischen, gesättigten oder ungesättigten $C_5$- bis $C_{18}$-Cycloalkylrest bedeuten und maximal zwei Reste gleichzeitig $C_1$-Alkyl sein dürfen,

Formel VI: $HNR^8R^9$

wobei $R^8$ und $R^9$ die oben angegebene Bedeutung haben und dabei maximal ein Rest $C_1$-Alkyl sein darf,

Formel VII: $NR^8R^9R^{10}R^{11}OH$

wobei $R^8$, $R^9$, $R^{10}$, $R^{11}$ gleich oder verschieden sein können und $R^8$, $R^9$, $R^{10}$ die oben genannte Bedeutung haben

und $R^{11}$ ebenfalls ein gesättigter oder ungesättigter $C_1$- bis $C_{18}$-Alkylrest oder ein aliphatischer, gesättigter oder ungesättigter $C_5$- bis $C_{18}$-Cycloalkylrest ist.

**7.** Verfahren nach Anspruch 6,
**dadurch gekennzeichnet,**
**dass**

| | |
|---|---|
| $R^1$ = | Acrylolyl, Methacryloyl, 2-Alkylacryloyl mit einer $C_2$- bis $C_6$-Alkylgruppe, Succinyl-, Maleinoyl-, Polyacryloyl-, der Säurerest eines Copolymeren aus Ethylen und Acrylsäure oder Hexamethylenbiscarbonyl, |
| $R^2$, $R^3$ = | n-Butyl, |
| $R^2$ + $R^3$ = | Säurerest der Bernsteinsäure, der Glutarsäure oder der Phthalsäure, |
| $R^5$ = | H und m = 11, |
| $R^8$, $R^9$, $R^{10}$ | in Formel V Ethyl, |
| $R^8$, $R^9$, $R^{10}$, $R^{11}$ | in Formel VII Methyl, Ethyl oder Propyl ist. |

**8.** Verfahren nach Anspruch 2,
**dadurch gekennzeichnet,**
**dass** als Base 3-Dodecyl-1-(2,2,6,6-Tetramethyl-4-piperidyl)pyrrolidin-2,5-dion, Diazabicyclooctan oder 4-Dimethylaminopyridin eingesetzt wird.

**9.** Verfahren nach einem der vorhergehenden Ansprüche,
**dadurch gekennzeichnet,**
**dass** 2-Aminoethanol und die Base während der Destillation gleichzeitig, nacheinander oder intermittierend dem Destillationssumpf zugefügt werden.

**10.** Verfahren nach einem der vorhergehenden Ansprüche,
**dadurch gekennzeichnet,**
**dass** 2-Aminoethanol und die Base während der Destillation gleichzeitig, nacheinander oder intermittierend der Rohstoffeinspeisung für die Destillation zugefügt werden.

**11.** Verfahren nach einem der vorhergehenden Ansprüche,
**dadurch gekennzeichnet,**
**dass** 2-Aminoethanol bezogen auf den Gehalt an Carbonylverbindung in einem Verhältnis von 1 bis 10 Moläquivalenten eingesetzt wird.

**12.** Verfahren nach einem der vorhergehenden Ansprüche,
**dadurch gekennzeichnet,**
**dass** 2-Aminoethanol bezogen auf den Gehalt an Carbonylverbindung in einem Verhältnis von 1,2 bis 5 Moläquivalenten, insbesondere von 1,5 bis 3 Moläquivalenten stehen.

**13.** Verfahren nach einem der vorhergehenden Ansprüche,
**dadurch gekennzeichnet,**
**dass** die Menge an Base in einem Anteil von 0,5 bis 5 Gew.-% bezogen auf die Einsatzmenge der Destillation eingesetzt wird.

**14.** Verfahren nach einem der vorhergehenden Ansprüche ,
**dadurch gekennzeichnet,**
**dass** die Menge an Base in einem Anteil von 1 - 4 Gew.-% bezogen auf die Einsatzmenge der Destillation eingesetzt wird.

**15.** Verfahren nach einem der vorhergehenden Ansprüche,
**dadurch gekennzeichnet**
**dass** während der Destillation eine Sumpftemperatur von 30 bis 100 °C, vorzugsweise von 50 bis 80 °C gewählt wird.

**16.** Verfahren nach einem der vorhergehenden Ansprüche,
**dadurch gekennzeichnet**

**dass** der Druck während der Destillation im Bereich von 100 mbar bis 5 bar liegt.

**17.** Verfahren nach einem der vorhergehenden Ansprüche,
**dadurch gekennzeichnet,**
**dass** der Druck während der Destillation im Bereich von 900 mbar bis 4 bar vorzugsweise bei Normaldruck liegt.

**18.** Verfahren nach einem der vorhergehenden Ansprüche,
**dadurch gekennzeichnet,**
**dass** die Destillation kontinuierlich oder diskontinuierlich durchgeführt wird.

**19.** Verfahren nach einem der vorhergehenden Ansprüche,
**dadurch gekennzeichnet,**
**dass** zur Destillation Gemische eingesetzt werden, die aus der Synthese von 2-Methoxypropen resultieren.

Europäisches Patentamt

# EUROPÄISCHER RECHERCHENBERICHT

Nummer der Anmeldung
EP 05 10 1847

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,X | DATABASE CA [Online] CHEMICAL ABSTRACTS SERVICE, COLUMBUS, OHIO, US; 3. September 1989 (1989-09-03), MACHO, VENDELIN ET AL: "Process for the production of 2-methoxypropene from petroleum cracking fractions" XP002344866 gefunden im STN Database accession no. 111:81215 | 1-3,19 | C07C41/58<br>C07C43/13 |
| Y | * Zusammenfassung * -& CS 259 389 B1 (CZECH.) 14. Oktober 1988 (1988-10-14) ----- | 1-5,9-19 | |
| D,Y | DE 11 34 076 B (E. MERCK AKTIENGESELLSCHAFT) 2. August 1962 (1962-08-02) | 1-5,9-19 | |
| A | Spalte 1, Zeilen 12-18, 40 - Spalte 2, Zeile 22; ----- | 6-8 | |

RECHERCHIERTE SACHGEBIETE (Int.Cl.7)

C07C

Der vorliegende Recherchenbericht wurde für alle Patentansprüche erstellt

| Recherchenort | Abschlußdatum der Recherche | Prüfer |
|---|---|---|
| München | 14. September 2005 | Kleidernigg, O |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

2

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 05 10 1847

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-09-2005

| Im Recherchenbericht angeführtes Patentdokument | Datum der Veröffentlichung | Mitglied(er) der Patentfamilie | Datum der Veröffentlichung |
|---|---|---|---|
| CS 259389 B1 | 14-10-1988 | CS 8604618 A1 | 15-03-1988 |
| DE 1134076 B | 02-08-1962 | KEINE | |

EPO FORM P0461

Für nähere Einzelheiten zu diesem Anhang : siehe Amtsblatt des Europäischen Patentamts, Nr.12/82